# EUROPEAN PATENT APPLICATION

(11) **EP 2 322 619 A1**
(43) Date of publication of application: **18.05.2011**
(21) Application number: 09176161.9
(22) Date of filing: 17.11.2009
(51) Int. Cl.: C12N 15/113, C07K 14/47

(54) **Inhibitors of centrosomal clustering**

(71) Applicant: Deutsches Krebsforschungszentrum, 69120 Heidelberg (DE)
(72) Inventor: Krämer, Alwin, 76149 Karlsruhe (DE); Leber, Blanka, 69221 Dossenheim (DE); Boutros, Michael, 69117 Heidelberg (DE); Fuchs, Florian, 69115 Heidelberg (DE); Maier, Bettina, 69115 Heidelberg (DE)
(74) Representative: Fiesser, Gerold Michael

(57) **Abstract**

The present invention is concerned with compounds modulating the function of a target gene in a cell, wherein the target gene is selected from the list consisting of CEP164, ARL2, HEI-C, SPC24, CENP-T, MKLP1, HIST1H2BO, HIST1H2BJ, FAM29A, ADPGK, ARSF, MAP6, GOLM1, STX5A, CACNA1F, TXNL1, USP54, RGL2, MAP4K5, TULP3, GRP137, ZNF354C, MEPCE, MTF2, TNRC4, POP1, SF3B1, SNRPD2, UPF3A, EIF3S6IP, ZNF17, NACAD, C4ORF15, C1ORF114, C19ORF52, ABHD8, TM4SF18, F8A1, KIAA1191, C20ORF149, and C110RF4. Also proposed is a medicament comprising said compound for the treatment or prevention of cancer. Furthermore, the use of said compound for the manufacture of a medicament for the treatment and / or prevention of cancer is proposed as well as methods for the identification of a compound capable of inhibiting ccntrosomal clustering, for the identification of a compound capable of binding to a polypeptide encoded by one of said target genes, and for the identification of cancer cells in a sample.

## Description

The present invention is concerned with compounds modulating the function of a target gene in a cell, wherein the target gene is selected from the list consisting of CEP164, ARL2, HEI-C, SPC24, CENP-T, MKLP1, HIST1H2B0, HISTIH2BJ, FAM29A, ADPGK, ARSF, MAP6, GOLM1, STX5A, CACNA1F, TXNL1, USP54, RGL2, MAP4K5, TULP3, GRP137, ZNF354C, MEPCE, MTF2, TNRC4, POP1, SF3B1, SNRPD2, UPF3A, EIF3S6IP, ZNF17, NACAD, C4ORF15, C1ORF114, C190RF52, ABHD8, TM4SF18, F8A1, KIAA1191, C20ORF149, and C11ORF4. Also proposed is a medicament comprising said compound for the treatment or prevention of cancer. Furthermore, the use of said compound for the manufacture of a medicament for the treatment and / or prevention of cancer is proposed as well as methods for the identification of a compound capable of inhibiting centrosomal clustering, for the identification of a compound capable of binding to a polypeptide encoded by one of said target genes, and for the identification of cancer cells in a sample.

Centrosomes are small organelles in metazoan (animal) cells consisting of two centrioles and a pericentriolar matrix. They play a central role in the formation of the mitotic spindle in that they are the microtubule-organizing centers during mitosis: Normal interphase cells contain only one centrosome per cell, which replicates during S-phase of the cell cycle to form the two centrosomes that migrate to the opposite poles of the cell, such that the mitotic spindle can form between the two centrosomes. Upon cell division, each daughter cell receives one centrosome. In recent years, it has been realized that cancer cells in almost all human malignancies, including lung, breast, prostate, brain, colon, and pancreas cancers as well as leukemias and lymphomas, contain supernumary centrosomes. Moreover, aberrant centrosome numbers have been correlated with chromosomal instability and clinical aggressiveness in many tumors.

The presence of supernumary centrosomes leads to the formation of multipolar spindles, which cause chromosomal mis-segregation and, as a consequence, apoptosis to occur. So, during tumorigenesis, cells are selected that employ a yet poorly defined mechanism to coalesce the existing centrosomes into two spindle poles ("centrosomal clustering"), ensuring proper chromosome distribution and, thus, survival of the cell.

Inhibition of centrosomal clustering by the drug Griseofulvin causes an increased frequency of multipolar mitoses, mitotic arrest and apoptosis in several different tumor cell lines, whereas normal cells are not affected. By inhibition of centrosomal clustering malignant cells are specifically inhibited in their proliferation while other dividing cells remain unaffected. This is in contrast to traditional chemo- and radiotherapy in cancer, where all fast-dividing cells are affected, leading to severe side-effects in traditional cancer therapy.

Results with the drug Griseofulvin are promising. However, the concentration of the drug needed in vitro to produce the effects is too high to make a future clinical use of this drug plausible. Therefore, there is still a need for means and methods to inhibit centrosomal clustering in cancer cells.

Moreover, diagnosis, staging, and prognosis evaluation of cancer samples still mainly rely on microscopical inspection of samples containing potential cancer cells, e.g. biopsies, by a pathologist. This evaluation is difficult to standardize, since it depends on the skill and experience of the pathologist. A further drawback is that this method cannot be automated. Accordingly, there is a need for well standardizable, automatable methods for cancer diagnosis, staging, and prognosis evaluation in patients.

The technical problem underlying the present invention, thus, could be seen as the provision of means and methods for complying with the aforementioned needs. The said technical problem is solved by the embodiments characterized in the claims and herein below.

Thus, the present invention relates to a compound modulating the function of a target gene in a cell, wherein the target gene is selected from the list consisting of CEP164, ARL2, HEI-C, SPC24, CENP-T, MKLP1, HIST1H2BO, HISTIH2BJ, FAM29A, ADPGK, ARSF, MAP6, GOLM1, STX5A, CACNA1F, TXNL1, USP54, RGL2, MAP4K5, TULP3, GRP137, ZNF354C, MEPCE, MTF2, TNRC4, POP1, SF3B1, SNRPD2, UPF3A, EIF3S6IP, ZNF17, NACAD, C4ORF15, C1ORF114, C19ORF52, ABHD8, TM4SF18, F8A1, KIAA1191, C20ORF149, and C11ORF4.

The term "compound" refers to a chemical molecule, i.e. any organic or inorganic substance. The organic molecule may belong to any known chemical class of molecules. Preferably, organic molecules are lipids, fatty acids, purines, pyrimidines, alkaloids, amino acids, peptides, polypeptides, proteins, biogenic amines, isoprenoids or steroids.

"Compound modulating the function of a target gene" refers to a compound that, when brought into contact with a cell, causes a change in the expression, activitiy, or stability of a target gene or its gene products. Preferably, said compound modulating the function of a target gene negatively interferes with, i.e. inhibits, the function of a target gene, meaning that it, preferably, decreases expression of said target gene or decreases the activity or stability of the product of said target gene. More preferably, said compound is selected from a list consisting of an RNAi agent, a ribozyme, a DNAzyme, an inhibitory antibody, and an aptamer. Methods of obtaining such compounds are well known in the art (see e.g. Bhindi et al. (2007), Am. J. Path. 171, 1079 - 1088). It is to be understood that modulating the function of a target gene refers to statistically significant modulations of the function, i.e. it can refer to modest changes in the function of a target gene, meaning a change in amount, activity, or half-life of 10 % or more, 20% or more, 30% or more, 40% or more, or 50 % or more. The extent of change appropriate will depend on the function that is to be modulated. E.g. in the case of inhibition of centrosomal clustering, inhibition of the function of one of the target genes to an extent leading to a fraction of at least 10%, at least 15%, at least 20%, at least 30%, at least 40%, at least 50% of cells undergoing non-bipolar division is appropriate. Whether modulation is statistically significant can be determined by the skilled artisan without further ado, preferably, by applying standard statistics such as, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001.

It is contemplated by the current invention that suitable compounds may be obtained by screening artificial chemical libraries obtained, e.g., by combinatorial chemistry approaches or by screening of natural compound libraries obtained, e.g., by fractioning extracts from biological organisms such as bacteria, funghi (e.g. Penicillium, Aspergillus), plants, or animals. Suitable compounds can also be generated by in silico screening methods based on, e.g., molecular modelling approaches.

Preferably, compounds modulating the function of a target gene are identified by using technologies such as microscopy (e.g. Laser scanning microscopy, fluorescence microscopy (including, but not limited to, scintillation proximity assays, time-resolved energy transfer fluorescence anisotropy, fluorescence correlation spectrocopy, and fluorescence fluctuation), nuclear-magnetic resonance, affinity chromatography, surface plasmon resonance, and DNA microarrays. More preferably, said technologies are used in high-throughput screening systems (see, for example, Liu et al. (2004), Am. J. Pharmacogenomics 4(4), 263 - 276).

The term "target gene" refers to a polynucleotide coding for an RNA selected from the list consisting of CEP164, ARL2, HEI-C, SPC24, CENP-T, MKLP1, HIST1H2BO, HIST1H2BJ, FAM29A, ADPGK, ARSF, MAP6, GOLM1, STX5A, CACNA1F, TXNL1, USP54, RGL2, MAP4K5, TULP3, GRP137, ZNF354C, MEPCE, MTF2, TNRC4, POP1, SF3B1, SNRPD2, UPF3A, EIF3S6IP, ZNF17, NACAD, C4ORF15, C1ORF14, SF3B1, SNRPD2, UPF3A, EIF3S6IP, ZNF17, NACAD, C4ORF15, C1ORF114, C19ORF52, ABHD8, TM4SF18, F8A1, KIAA1191, C20ORF149, and C11ORF4.

Preferably, said RNAs are encoded, respectively, by the genes CEP164 (Genbank Acc. No.: NM_014956.4, GI:115648141), ARL2 (Genbank Acc. No.:NM_001667.2, GI:14861288), HEI-C (Genbank Acc. No.: NM_138443.3, GI:224586869), SPC24 (Genbank Acc. No.: NM_182513.2, GI:148747229), CENP-T (Genbank Acc. No.: NM_025082.3, GI:126722968), MKLP1 (Genbank Acc. No.: NM_004856.4, GI:20143965), HIST1H2BO (Genbank Acc. No.: NM_003527.4, GI:16306565), HIST1H2BJ (Genbank Acc. No.: NM_021058.3, GI:20336753), FAM29A (Genbank Acc. No.: NM_017645.3, GI:31377561), ADPGK (Genbank Acc. No.: NM_031284.4, GI:187829199), ARSF (Genbank Acc. No.: NM_004042.3, GI:31742481), MAP6 (Genbank Acc. No.: NM_024871.2, GI:209915605), GOLM1 (Genbank Acc. No.: NM_016548.2, GI:29550837), STX5A (Genbank Acc. No.: NM_003164.3, GI:94400931), CACNA1F (Genbank Acc. No.: NM_005183.2, GI:53832006), TXNL1 (Genbank Acc. No.: NM_031284.4, GI:187829199), USP54 (Genbank Acc. No.: NM_152586.3, GI:124001557), RGL2 (Genbank Acc. No.: NM_004761.2, GI:21361071), MAP4K5 (Genbank Acc. No.: NM_006575.3, GI:38570133), TULP3 (Genbank Acc. No.: NM_003324.3, GI:42544229), GRP137 (Genbank Acc. No.: NM_173481.2, GI:34222226), ZNF354C (Genbank Acc. No.: NM_014594.1, GI:30794503), MEPCE (Genbank Acc. No.: NM_019606.4, GI:47271405), MTF2 (Genbank Acc. No.: NM_007358.3, GI:166706893), TNRC4 (Genbank Acc. No.: NM_007185.3, GI:71164893), POP1 (Genbank Acc. No.: NM_015029.2, GI:225007647), SF3B1 (Genbank Acc. No.: NM_012433.2, GI:54112116), SNRPD2 (Genbank Acc. No.: NM_004597.4, GI:29294622), UPF3A (Genbank Acc. No.: NM_023011.2, GI:18375523), EIF3S6IP (Genbank Acc. No.: NM_016091.2, GI:51093838), ZNF17 (Genbank Acc. No.: NM_006959.2, GI:115494999), NACAD (Genbank Acc. No.: XM_166571.6, GI:169170976), C4ORF15 (Genbank Acc. No.: NM_024511.5, GI:141802706), C1ORF114 (Genbank Acc. No.: NM_021179.1, GI:10880974), C19ORF52 (Genbank Acc. No.: NM_138358.2, GI:34328079), ABHD8 (Genbank Acc. No.: NM_024527.4, GI:145611433), TM4SF18 (Genbank Acc. No.: NM_138786.2, GI:142345050), F8A1 (Genbank Acc. No.: NM_012151.3, GI:56790933), KIAA1191 (Genbank Acc. No.: NM_020444.3, GI:119120886), C20ORF149 (Genbank Acc. No.: NM_024299.2, GI:34147371), and C11ORF4 (Genbank Acc. No.: NM_020155.2, GI:21361824) (see also table 1).

Moreover, the term "target gene" as used in accordance with the present invention further encompasses variants of the aforementioned specific genes which comprise nucleic acid sequences at least 75%, at least 80%, at least 85%, at least 90%, at least 95%, at least 98% or at least 99% identical to the target genes above. The percent identity values are, preferably, calculated over the entire nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981)), which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000.

Target genes also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 × sodium chloride/sodium citrate (= SSC) at approximately 45°C, followed by one or more wash steps in 0.2 × SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 × SSC (pH 7.2). If organic solvent is present in the above mentioned buffer, for example 50% formamide, the temperature under standard conditions is approximately 42°C. The hybridization conditions for DNA:DNA hybrids are preferably for example 0.1 × SSC and 20°C to 65°C, preferably between 30°C and 45°C. The hybridization conditions for DNA:RNA hybrids are preferably, for example, 0.1 × SSC and 30°C to 55°C, preferably between 40°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, variants of target genes are obtainable by PCR-based techniques such as mixed oligonucleotide primer- based amplification of DNA, i.e. using degenerated primers against conserved sequences of the genes of the present invention. Conserved sequences of the target genes of the present invention may be identified by a sequence comparison of the nucleic acid sequence of the target genes with sequences of homologs from other species. As a template, DNA or cDNA from animals may be used.

Moreover, target genes further encompasses variants of the aforementioned specific genes which may represent orthologs, paralogs or other homologs of the polynucleotide of the present invention. The variants, preferably, comprise a nucleic acid sequence characterized in that the sequence can be derived from the aforementioned specific gene sequences described above by at least one nucleotide substitution and/or addition and/or deletion.

The target genes of the present invention either essentially consist of the aforementioned nucleic acid sequences or comprise the aforementioned nucleic acid sequences. Thus, they may contain further nucleic acid sequences as well. Specifically, the variants may encode fusion proteins wherein one partner of the fusion protein is a polypeptide encoded by a target gene or a variant recited above. Such fusion proteins may comprise as additional part polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and comprise FLAG-tags, 6-histidine-tags, MYC-tags and the like.

All target genes still encode a polypeptide having at least one, preferably all, biological and / or immunological properties of the polypeptides encoded by the aforementioned specific genes. More preferably, the biological property retained by target genes is the ability to mediate centrosomal clustering; Suitable assays for assessing the activity of gene products in centrosomal clustering are described in the accompanying Examples or in Rebacz et al., Identification of griseofulvin as an inhibitor of centrosomal clustering in a phenotype-based screen. Cancer Research 67: 6342-6350, 2007. Most preferably, the biological property retained is the function of the gene product in mitosis (CEP164, ARL2, HEI-C, SPC24, CENP-T, MKLP1, HIST1H2BO, HIST1H2BJ, FAM29A), in signal transduction (RGL2, MAP4K5, TULP3, GRP137) or in transcription and / or translation (ZNF354C, MEPCE, MTF2, TNRC4, POP1, SF3B1, SNRPD2, UPF3A, EIF3S6IP).

Preferably, silencing of the target gene causes at least 10% of cells to undergo multipolar division (CEP164, ARL2, HEI-C, HISTIH2BJ, STX5A, RGL2, MAP4K5, GRP137, ZNF354C, MTF2, SNRPD2, UPF3A, EIF3S6IP, C19ORF52, ABHD8, TM4SF18, F8A1, KIAA1191, C20ORF149, and C11ORF4), more preferably silencing of the target gene causes at least 15% of cells to undergo multipolar division, (SPC24, HIST1H2BO, ADPGK, ARSF, MAP6, GOLM1, CACNA1F, TXNL1, USP54, TULP3, MEPCE, TNRC4, POP1, SF3B1, ZNF17, and C10RF114), and most preferably silencing of the target gene causes at least 20% of cells to undergo multipolar division (CENP-T, MKLP1, FAM29A, NACAD, and C4ORF15). It is to be understood, however, that the fraction of cells undergoing multipolar division depends an various parameters, e.g. cell type and tissue under evaluation, the molecular nature of the compound, mode of transfer of the compound into the cell, and others.

A "cell" in the context of the present invention is a metazoan (animal) cell comprising supernumary centrosomes; preferably, said cell is a mammalian cell, more preferably a human cell, and most preferably a human cancer cell.

"RNA interference (RNAi)" refers to sequence-specific, post-transcriptional gene silencing of a selected target gene by degradation of RNA transcribed from the target gene (target RNA) or by inhibition of translation of said target RNA. Target RNAs preferably are mRNAs, however, any type of RNA is encompassed by the RNAi methods of the invention. It is to be understood that silencing as used herein does not necessarily mean the complete abolishment of gene expression in all cases. RNAi, preferably, reduces gene expression by at least 50%, at least 60%, at least 70%, at least 80%, at least 90%, at least 95%, at least 98%, or at least 99% as compared to the expression level in a reference without RNAi.

RNAi requires in the cell the presence of dsRNAs that are homologous in sequence to the target RNAs. The term "dsRNA" refers to RNA having a duplex structure comprising two complementary and anti-parallel nucleic acid strands. The RNA strands forming the dsRNA may have the same or a different number of nucleotides, whereby one of the strands of the dsRNA can be the target RNA. It is, however, also contemplated by the present invention that the dsRNA is formed between two sequence stretches on the same RNA molecule.

RNAi may be used to specifically inhibit expression of the target genes of the present invention in vivo. Accordingly, it may be used for therapeutic approaches to treat cancers which are accompanied with an altered expression of the target genes of the present invention, e.g., an enhanced expression in cancer cells or an expression in compartments or cells where the genes are not normally expressed. For such therapeutic approaches, expression constructs for siRNA may be introduced into target cells of the host which suffer from altered target gene expression. Accordingly, siRNA may be combined efficiently with other therapy approaches.

Methods relating to the use of RNAi to silence genes in animals, including mammals, are known in the art (see, for example, Hammond et al. (2001), Nature Rev. Genet. 2, 110-119; Bernstein et al. (2001), Nature 409, 363-366; WO 9932619; and Elbashir et al. (2001), Nature 411: 494-498).

As used herein, the term "RNAi agent" refers to either a siRNA agent or a miRNA agent as specified below. The RNAi agent of the present invention is of sufficient length and complementarity to stably interact with the target RNA, i.e. it comprises at least 15, at least 17, at least 19, at least 21, at least 22 nucleotides complementary to the target RNA. By "stably interact" is meant interaction of the RNAi agent or its products produced by the cell with a target RNA, e.g., by forming hydrogen bonds with complementary nucleotides in the target RNA under physiological conditions.

Not all nucleotides of an RNAi agent necessarily exhibit complete Watson-Crick base pairs in the interaction with the target RNA; the two RNA strands may be substantially complementary. Preferably, complementarity between the RNAi agent and the RNA target is 100%, but can be less if desired, preferably 91%, 92%, 93%, 94%, 95%, 96%, 97%, 98%, or 99%. For example, 19 bases out of 21 bases may be base-paired. In some instances, where distinction between various allelic variants is desired, 100% complementarity to the target gene may be required in order to effectively discern the target sequence from the other allelic sequence. When selecting between allelic targets, choice of length is also an important factor because it is the other factor involved in the percent complementary and the ability to differentiate between allelic differences.

The term "siRNA agent" as meant herein ecompasses: a) a dsRNA consisting of at least 15, at least 17, at least 19, at least 21 consecutive nucleotides base-paired, i.e. forming hydrogen bonds with complementary nucleotides. b) a small interfering RNA (siRNA) molecule or a molecule comprising an siRNA molecule. The siRNA is a single-stranded RNA molecule with a length, preferably, greater than or equal to 15 nucleotides and, preferably, a length of 15 to 49 nucleotides, more preferably 17 to 30 nucleotides, and most preferably 17, 18, 19, 20, 21, 22, 23, 24, 25, 26, 27, 28, 29, and 30 nucleotides. c) a polynucleic acid encoding a) or b), wherein, preferably, said polynucleic acid is operatively linked to an expression control sequence. Thus, the function of the siRNA agent to inhibit expression of the target gene can be modulated by said expression control sequence. Preferred expression control sequences are those which can be regulated by exogenous stimuli, e.g. the tet operator, whose activity can be regulated by tetracycline, or heat inducible promoters. Alternatively or in addition, one or more expression control sequences can be used which allow tissue-specific expression of the siRNA agent.

It is, however, also contemplated by the current invention that the RNAi agent is a miRNA agent. A "miRNA agent" as meant herein encompasses: a) a pri-microRNA, i.e. an mRNA comprising at least 30, at least 40, at least 50, at least 60, at least 70 nucleotides base-paired to a complemetary sequence on the same mRNA molecule ("stem"), i.e. as a dsRNA, separated by a strech of non-base-paired nucleotides ("loop"). b) a pre-microRNA, i.e. a dsRNA molecule comprising a stretch of at least 19, at least 20, at least 21, at least 22, at least 23, at least24, at least 25 base-paired nucleotides formed by nucleotides of the same RNA molecule (stem), separated by a loop. c) a microRNA (miRNA), i.e. a dsRNA comprising at least 15, at least 17, at least 18, at least 19, at least 21 nucleotides on two separate RNA strands. d) a polynucleic acid encoding a) or b), wherein, preferably, said polynucleic acid is operatively linked to an expression control sequence as specified above.

As used herein, the term "ribozyme" refers to an RNA molecule specifically hybridizing to a target RNA molecule and catalysing the hydrolysis of one or more phosphodiester bonds in said target RNA molecule, causing the target RNA to be degraded by cellular enzymes. RNA sequences showing suitable catalytic properties, like hammerhead ribozyme, hairpin ribozyme, or RNase P are known in the art (see, e.g. Doherty and Doudna (2001), Annu. Rev. Biophys. Biomol. Struct. 30, 457 - 475). Sequence specificity and, thus, target RNA specificity is accomplished by specific binding of the ribozyme to the target RNA by means of Watson-Crick base pairing of complementary, anti-parallel RNA strands. Methods of generating ribozymes directed against RNA sequences of interest are known in the art (see, for example, Citti and Rainaldi (2005), Curr. Gene Ther. 5(1), 11 - 24).

The term "DNAzyme" refers to a single-stranded DNA molecule having the same binding and catalytic properties as a ribozyme, however, said DNAzyme comprises desoxyribonucleotides instead of ribonucleotides. Methods of generating DNAzymes, like in vitro selection, are known to the one skilled in the art (see, e.g. Achenbach et al. (2004) Curr. Pharm. Biotechnol. 5(4), 321 - 336).

It is, however, also contemplated by the current invention that the ribozyme or DNAzyme comprises modified nucleotides or compounds modifying the stability, specificity, or catalytic properties of said ribozymes or DNAzymes. It is to be understood that "catalysing" as used herein does not necessarily mean the promotion of more than one hydrolysis event per molecule of ribozyme or DNAzyme.

The term "antibody" as used in this specification refers to a molecule from the subgroup of gamma globulin proteins which is also referred to as the immunoglobulins (Ig). Antibodies can, preferably, be of any subtype, i.e. IgA, IgD, IgE, IgM or, more preferably, IgG. Antibodies against polypeptides encoded by target genes of the invention can be prepared by well known methods using a purified polypeptide or a suitable fragment derived therefrom as an antigen. A fragment which is suitable as an antigen may be identified by antigenicity determining algorithms well known in the art. Such fragments may be obtained either by proteolytic digestion from polypeptides encoded by target genes or may be synthetic peptides. Preferably, the antibody of the present invention is a monoclonal antibody, a polyclonal antibody, a single chain antibody, a human or humanized antibody or primatized, chimerized or fragment thereof. Also comprised as antibodies by the present invention are a bispecific antibody, a synthetic antibody, an antibody fragment, such as Fab, Fv or scFv fragments etc., or a chemically modified derivative of any of these. An antibody of the present invention preferably binds specifically (i.e. does not cross react with other polypeptides or peptides) to one of the polypeptides of the invention. Specific binding can be tested by various well known techniques.

Antibodies or fragments thereof can be obtained by using methods which are described, e.g., in Harlow and Lane "Antibodies, A Laboratory Manual", CSH Press, Cold Spring Harbor, 1988. Monoclonal antibodies can be prepared by the techniques originally described in Köhler and Milstein, Nature 256 (1975), 495, and Galfré, Meth. Enzymol. 73 (1981), 3, which comprise the fusion of mouse myeloma cells to spleen cells derived from immunized mammals.

Antibodies can preferably be used for the inhibition or for the monitoring of the presence of the polypeptides encoded by the target genes according to the present invention. Said monitoring can be performed, for example, in recombinant organisms or in cells.

The term "inhibitory antibody" relates to an antibody inhibiting a polypeptide encoded by the target gene referred to in accordance with the present invention. Said inhibition preferably is caused by binding of the inhibitory antibody to an active center or to an interaction site of a polypeptide of the invention, causing a decrease of centrosomal clustering in the cell treated with said inhibitory antibody. The person skilled in the art knows means and methods to obtain inhibitory antibodies to specific proteins, like e.g. the method proposed by Rosen and Koshland (1988), Anal. Biochem. 170(1), 31 - 37. It is to be understood that inhibiting as used herein does not necessarily mean the complete abolishment of activity in all cases. Inhibitory antibodies, preferably, reduce centrosomal clustering by at least 10%, at least 20%, at least 30%, at least 40%, or at least 50% as compared to a reference.

In the context of this invention, an "aptamer" is an oligonucleic acid or a peptide specifically binding to a polypeptide encoded by one of the target genes of the present invention. Peptide aptamers, preferably, are peptides comprising 8-80 amino acids, more preferably 10-50 amino acids, and most preferably 15-30 amino acids. They can e.g. be isolated from randomized peptide expression libraries in a suitable host system like baker's yeast (see, for example, Klenenz et al. (2002), Cell. Mol. Life Sci. 59, 1993 - 1998). Peptide aptamers, preferably, are used as free peptides; it is, however, also contemplated by the present invention that peptide aptamers are fused to proteins serving as "scaffolds", meaning that the covalent linking to said proteins serves to fix the three-dimensional structure of said peptide aptamer to one specific conformation. An RNA or DNA aptamer is an RNA or DNA molecule that is able to specifically bind to the three-dimensional surface of a polypeptide and to inhibit the function of said polypeptide. RNA or DNA aptamers can be obtained e.g. by in vitro selection, e.g. systematic evolution of ligands by exponential enrichment (SELEX). Methods relating to the development and use of RNA and DNA aptamers are known in the art (see, for example, Ulrich (2006), Handb. Exp. Pharmacol. 173, 305 - 326 and Ulrich (2005), Med. Chem. 1(2), 199 - 208).

"Centrosomal clustering" as meant herein relates to the competence of cells containing supernumerary centrosomes to coalesce all centrosomes on the two poles of cell division, thus avoiding multipolar cell division and subsequent cell death. "Mediation of centrosomal clustering" relates to the relevance of the presence of a polypeptide encoded by one of the target genes for the cell's competence to achieve centrosomal clustering. The relevance of the presence of a polypeptide for centrosomal clustering can be e.g. assessed by the method of Example 2, i.e. by depleting the cell of the polypeptide of interest and determining the number of cells undergoing multipolar division instead of bipolar division and by comparing this number to a control without depletion of the polypeptide of interest. It is to be understood that negative interference with or inhibition of centrosomal clustering as used herein does not necessarily mean the complete abolishment of centrosomal clustering in all cases. Preferably, the number of cells undergoing non-bipolar cell division under inhibiting conditions is at least 10%, at least 20%, at least 30%, at least 40%, or at least 50% as compared to a reference.

The definitions made above apply *mutatis mutandis* to the following:

In another preferred embodiment, the current invention relates to a medicament comprising a compound modulating the function of a target gene as specified above for the treatment or prevention of cancer.

The term "medicament" as used herein comprises the compounds of the present invention and optionally one or more pharmaceutically acceptable carrier. The compounds of the present invention can be formulated as pharmaceutically acceptable salts. Acceptable salts comprise acetate, methylester, HCl, sulfate, chloride and the like. The medicaments are, preferably, administered topically or systemically. Suitable routes of administration conventionally used for drug administration are oral, intravenous, or parenteral administration as well as inhalation. However, depending on the nature and mode of action of a compound, the medicaments may be administered by other routes as well. For example, polynucleotide compounds may be administered in a gene therapy approach by using viral vectors or viruses or liposomes.

Moreover, the compounds can be administered in combination with other drugs either in a common medicament or as separated medicaments wherein said separated medicaments may be provided in form of a kit of parts.

The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are selected so as not to affect the biological activity of the combination. Examples of such diluents are distilled water, physiological saline, Ringer's solutions, dextrose solution, and Hank's solution. In addition, the medicament or formulation may also include other carriers, adjuvants, or nontoxic, nontherapeutic, nonimmunogenic stabilizers and the like.

A therapeutically effective dose refers to an amount of the compounds to be used in a medicament of the present invention which prevents, ameliorates or treats the symptoms accompanying cancers referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and by clinical factors, preferably in accordance with any one of the above described methods. As is well known in the medical arts, dosage for any one patient depends upon many factors, e.g. the patient's size, body surface area, age, the particular compound to be administered, sex, time and route of administration, general health, and other drugs being administered concurrently. A typical dose can be, for example, in the range of 0.01 to 1 mg / kg body mass; however, doses below or above this exemplary range are envisioned, especially considering the aforementioned factors. Depending on the compound, the subject and the mode of administration, the quantity of substance administration may vary over a wide range to provide from about 0.001 mg per kg body mass to about 10 mg per kg body mass, preferably. Progress can be monitored by periodic assessment.

The medicaments and formulations referred to herein are administered at least once in order to treat or ameliorate or prevent a disease or condition recited in this specification. However, the said medicaments may be administered more than one time, for example from one to four times daily up to a non-limited number of days.

Specific medicaments are prepared in a manner well known in the pharmaceutical art and comprise at least one active compound referred to herein above in admixture or otherwise associated with a pharmaceutically acceptable carrier or diluent. For making those specific medicaments, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or users' instructions in order to anticipate dose adjustments depending on the considered recipient.

The term "treatment" refers to amelioration of the diseases (cancers) referred to herein or of the symptoms accompanied therewith to a significant extent. Said treatment as used herein also includes an entire restoration of the health with respect to the diseases referred to herein. It is to be understood that treatment as used in accordance with the present invention may not be effective in all subjects to be treated. However, the term shall require that a statistically significant portion of subjects suffering from a disease referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.

The tem "prevention" refers to retainment of health with respect to the diseases (cancers) referred to herein for a certain period of time in a subject. It will be understood that the said period of time is dependent on the amount of the drug compound which has been administered and individual factors of the subject. It is to be understood that prevention may not be effective in all subjects treated with the compound according to the present invention. However, the term requires that a statistically significant portion of subjects of a cohort or population are effectively prevented from suffering from a disease referred to herein or its accompanying symptoms. Preferably, a cohort or population of subjects is envisaged in this context which normally, i.e. without preventive measures according to the present invention, would develop a disease as referred to herein. Whether a portion is statistically significant can be determined without further ado by the person skilled in the art using various well known statistic evaluation tools discussed above. Preferably, prevention shall be effective for at least 60%, at least 70%, at least 80%, or at least 90% of the subjects of a given cohort or population.

"Cancer" in the context of this invention refers to a disease of an animal, including man, characterized by uncontrolled growth by a group of body cells ("cancer cells"). This uncontrolled growth may be accompanied by intrusion into and destruction of surrounding tissue and possibly spread of cancer cells to other locations in the body.

Preferably, the cancer is selected from the list consisting of acute lymphoblastic leukemia, acute myeloid leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor. More preferably, the cancer is a myeloma, a leukemia, a colon cancer, a cervix carcinoma, a pancreatic cancer, a breast cancer, a glioblastoma, or an osteosarcoma; most preferably, the cancer is a head and neck cancer.

Another preferred embodiment of the current invention is the use of a compound modulating the function of a target gene as specified above for the manufacture of a medicament for the treatment or prevention of cancer.

In another preferred embodiment, the current invention relates to the use of a polypeptide encoded by a target gene selected from the list defined above for the manufacture of a medicament for the treatment or prevention of cancer.

The term "polypeptide" as used herein refers to amino acid chains of between 50 to 1000 amino acids in length comprising the amino acid sequences encoded by the target genes as specified above or partial sequences thereof. The polypeptide of the present invention can be recombinantly manufactured or may be chemically synthesised. The polypeptide may comprise further amino acids which may serve as a tag for purification or detection. Moreover, the polypeptide of the present invention may be comprised by a fusion polypolypeptide.

In a preferred embodiment of the polypeptide of the present invention, said polypeptide further comprises a detectable tag. The term "detectable tag" refers to a stretch of amino acids which are added to or introduced into the polypeptide of the invention. Preferably, the tag shall be added C- or N- terminally to the polypeptide of the present invention. The said stretch of amino acids shall allow for detection of the polypeptide by an antibody which specifically recognizes the tag or it shall allow for forming a functional conformation, such as a chelator or it shall allow for visualization by fluorescent tags. Preferred tags are the Myc-tag, FLAG-tag, 6-His-tag, HA-tag, GST-tag or GFP-tag. These tags are all well known in the art.

Preferably, polypeptides of the present invention include variants of said amino acid sequences, said variants having an amino acid sequence being at least 70%, at least 80%, at least 90%, at least 95%, least 96%, at least 97%, at least 98% or at least 99% identical to the amino acid sequence of the polypeptide provided, however, that the said variant retains the function of said polypeptide. The degree of identity between two given sequences (amino acids or nucleic acids) can be determined as specified above.

The term polypeptide also includes chemically modified polypeptides, e.g., polypeptides containing modified amino acids or polypeptides which are, e.g., biotinylated, or are coupled to fluorophores, such as fluorescin, or Cy 3, are conformationally restricted, e.g. by disulfide bridging or by stapling (Walensky 2004, Science 305(5689): 1466-1470), or are linked to cell penetration polypeptides or protein transduction domains (Snyder 2004, Pharm Res 21(3): 389-393). Such modifications may improve the biological properties of the polypeptides, e.g., cell penetration, binding, stability, or may be used as detection labels.

The variant or modified polypeptides, preferably, retain the biological activity of the polypeptides. The conservation of activity can be tested by the one skilled in the art by testing if the variant is able to complement a disabling mutation in or silencing of the gene coding for the polypeptide. E.g. it is feasible for the person skilled in the art to perform a silencing experiment as outlined in example 2 and, in a parallel experiment, to test if over-expression of said variant can decrease the fraction of cells undergoing non-bipolar division to normal values. It is to be understood that a complete reversion to control values can not be expected in all cases, so a decrease of the fraction of cells undergoing non-bipolar division to at most 50%, at most 40%, at most 30%, at most 20%, or at most 10% of values obtained for cells grown under silencing conditions shall be considered indicative of complementation and, thus, conservation of activity.

It is also contemplated by the present invention that the medicament manufactured comprising polypeptides encoded by the target genes of this invention is a vaccine.

The term "polynucleotide" as used in accordance with the present invention relates to a polynucleotide comprising a nucleic acid sequence of a target gene or one of its variants as specified above, coding for a polypeptide crucial for centrosomal clustering.

The polynucleotide of the invention, preferably, is operatively linked to expression control sequences allowing expression in metazoan cells or isolated fractions thereof. Expression of said polynucleotide comprises transcription of the polynucleotide, preferably into a translatable mRNA. Regulatory elements ensuring expression in metazoan cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Possible regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Moreover, inducible expression control sequences may be used. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide.

The polynucleotide of the present invention shall be provided, preferably, either as an isolated polynucleotide (i.e. isolated from its natural context) or in genetically modified form. The polynucleotide, preferably, is RNA or DNA, including cDNA. The term encompasses single as well as double stranded polynucleotides. Moreover, comprised are also chemically modified polynucleotides including naturally occurring modified polynucleotides such as glycosylated or methylated polynucleotides or artificially modified ones such as biotinylated polynucleotides.

In another preferred embodiment, the current invention relates to a method for the identification of a compound capable of inhibiting the function of a target gene in centrosomal clustering, comprising the steps of
a) expressing in a target cell a polynucleotide comprising a target gene selected from the group defined in claim 1,
b) contacting said target cell with a compound suspected of being able to inhibit the function of said target gene, and
c) determining the number of centrosomal clusters in said target cell, whereby a decrease in the number of centrosomal clusters after over-expression of said target gene is indicative for a compound capable of inhibiting the function of said target gene in centrosomal clustering.

The method of the present invention, preferably, is an *in vitro* method. Moreover, it may comprise steps in addition to those explicitly mentioned above. For example, further steps may relate to sample pre-treatments or evaluation of the results obtained by the method. The method may be carried out manually or assisted by automation. Preferably, one or more steps may in total or in part be assisted by automation, e.g. by suitable robotic and sensory equipment for the determination in the last step.

As used herein, the term "compound capable of inhibiting centrosomal clustering" relates to a compound bearing the ability to reduce the cell's competence to achieve centrosomal clustering. As a consequence of the inhibition of centrosomal clustering, the cell contains more than two mitotic spindle poles. The fraction of cells containing more than two mitotic spindle poles can be determined as specified in Example 2. Preferably, the fraction of cells containing more than two mitotic spindles in the presence of such compound is at least 10%, at least 20%, at least 30%, or at least 40%.

The term "target cell", preferably, relates to a cell containing supernumerary centrosomes maintained in vitro in a suitable cultivation medium, where "maintaining" relates to incubating a target cell under conditions suited for the growth of the respective target cell, which vary with the type of host cell and which are well known in the art (see, for example, Example 1).

The term "contacting" as used in the context of the method of the present invention is understood by the skilled person. Preferably, the term relates to bringing a compound of the present invention into physical contact with a target cell or a polypeptide and thereby allowing the compound and the target cell or polypeptide to interact.

In another preferred embodiment, the current invention relates to a method for the identification of a compound capable of binding to a polypeptide encoded by a target gene as defined in above comprising the steps of
a) contacting said polypeptide with a compound suspected to bind to said polypeptide and
b) determining the amount of said compound bound to said polypeptide, whereby an increased binding compared to a reference is indicative for a compound capable of binding to said gene product.

As used in this specification, the term "compound capable of binding to a polypeptide" relates to a compound being able to physically interact with a polypeptide of this invention. Methods to determine the amount of a compound bound to a polypeptide are well known in the art and include, but are not limited to, e.g., equilibrium dialysis, south-western blotting or plasmon surface resonance measurement. Preferably, said interaction leads to an accumulation of said compound in the vincinity of said polypeptide. More preferably, said interaction causes a decrease in the ability of said polypeptide to mediate centrosomal clustering. Preferably, accumulation of said compound can be detected by visible or microscopical inspection either as a result of the physical properties of said compound or after chemical modification of said compound, e.g. conjugation to a colored or color-producing compound. More preferably, said compound is an antibody.

In a further preferred embodiment, the present invention relates to a method for the identification of cancer cells in a sample comprising the steps of
a) contacting said sample with a diagnostic antibody directed against a polypeptide encoded by the polynucleotide as defined in claim 10 and
b) determining the amount of diagnostic antibody bound to said sample, whereby an increased binding compared to a reference is indicative for a cancer cell.

"Identification of cancer cells" as used herein relates to assessing the presence or absence of cancer cells in a sample. Cancer cells can preferably be identified based on a visual distinction, e.g. by staining cancer cells but not normal cells, e.g. by immunostaining methods well known in the art, followed by microscopical or macroscopical inspection. More preferably, identification can also be achieved by means of a color-producing reaction like, e.g., in an enzyme.linked immunosorbent assay.

The term "sample", preferably, relates to a specimen taken from a part of the body of an animal. Preferably, said animal is a mammal and most preferably said animal is a human. The methods for obtaining a specimen from the body of an animal are well known in the art. Preferably, specimens are obtained by removing small amounts of cells from the body, most preferably by excision or by taking a smear or a swab or plugging hairs. Preferably, the sample may have any volume and concentration deemed appropriate. Moreover the sample may have been further processed in order to carry out the method of the present invention. Preferably, polynucleotides such as DNA or RNA, preferably DNA, or proteins might be extracted and / or purified from the sample by methods and means known in the art. Thus, the term sample may also relate to polynucleotides, preferably DNA, or protein(s) extracted and /or purified from any sample as mentioned above.

However, it is also contemplated by the present invention that the sample may be a cell culture sample, relating to a sample from cells which are maintained in vitro in a suitable cultivation medium. Preferably, said cells are animal cells, more preferably mammalian cells and most preferably human cells. The sample may be or be derived from cells or from cell culture supernatant which may have been processed as referred to above.

A "diagnostic antibody" as used in this specification relates to an antibody directed against one of the polypeptides of this invention as specified above, said polypeptide being more abundant in cancer cells as compared to non-cancer cells. Methods to assess the relative abundance of a protein in a cell are known to the person skilled in the art and include, but are not limited to, western blotting, immune precipitation, and determination of enzymatic activity. Preferably, said diagnostic antibody binds specifically, i.e. binds essentially without cross-reacting with other proteins.

In a further preferred embodiment, the current invention relates to a method for the identification of cancer cells in a sample comprising the steps of
a) contacting said sample with a diagnostic polynucleotide being capable of specifically hybridizing to the messenger RNA transcribed from a target gene selected from the list defined in claim 1 and
b) determining the amount of said polynucleotide bound to said sample, whereby an increased binding compared to a reference is indicative for a cancer cell.

The term "diagnostic polynucleotide" as used herein relates to a polynucleotide specifically hybridizing to a transcript from a target gene as specified above, wherein said target gene is expressed to a higher extent in cancer cells. A person skilled in the art is aware of methods to assess the relative abundance of RNA in a cell. Said methods include, but are not limited to, northern blotting, RT-qPCR and alike.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Figure Legends

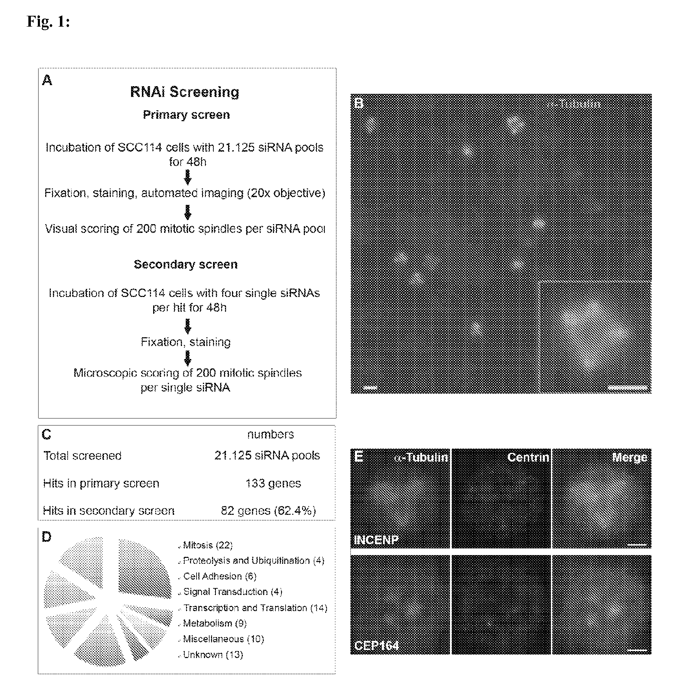

Fig. 1. Genome-wide screen for genes required for clustering of supernumerary centrosomes in SCC114 cells. (A) Scheme of genome-wide RNAi screen in SCC114 cells. The procedures for the primary and secondary screen are given. (B) Sample image from screen. Multipolar spindles after treatment with aurora-B-specific siRNA are shown. Cells were stained for microtubules (α-tubulin, green) and DNA (DAPI, blue). A multipolar spindle is shown enlarged in an inset. (C) Table summarizing the screening results. (D) Gene ontology annotations for 82 genes from the screen. (E) Multipolar mitoses resulting from siRNA-mediated knockdown of INCENP and CEP164, respectively. Cells were stained for microtubules (α-tubulin, green), centrioles (centrin, red), and DNA (DAPI, blue). Bar, 5 µm

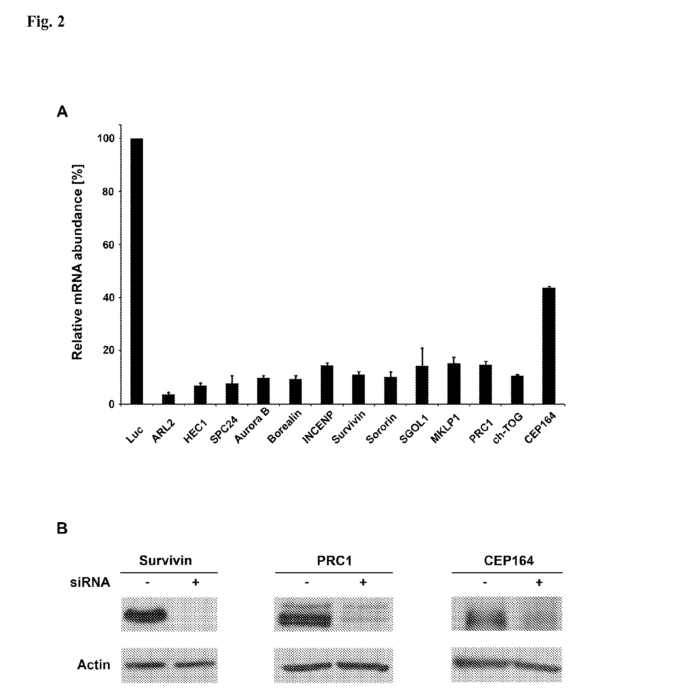

Fig. 2. siRNA-mediated knockdown efficacy of randomly chosen screening hits in SCC114 cells.

### Examples

### Example 1: Materials and Methods

### RNAi Screen

For the primary screen siGENOME SMART pools (Dharmacon) were transferred into 384-well plates. After incubation with the transfection reagent (Dharmafect-1), SCC114 cells were added to the mixture of siRNA and transfection reagent using an automated Multidrop Combi dispensing system (Thermo Scientific). After 48 hours the cells were stained with a mouse monoclonal antibody to α-tubulin (DM1A, Sigma) followed by an anti-mouse Alexa 488 antibody (Molecular Probes, Invitrogen) and Hoechst 33342 (Invitrogen). All steps were done by a Biomek FX Laboratory Automation Workstation. Images for the evaluation of the primary screen were taken using an automated BD Pathway 855 bioimaging system (Becton Dickinson) equipped with a 20x 0.75 NA objective (Olympus) and a monochrome digital black and white camera (Orca-ER, Hamamatsu).

### Cell Culture

SCC114 cells were cultured in DMEM (Gibco) supplemented with 10% FCS (Biochrom AG). SCC114 cells stably expressing green fluorescent protein (GFP)-α-tubulin were maintained under selective pressure by the addition of geniticin (PAA Laboratories).

### Antibodies

The following antibodies were used: mouse monoclonal antibodies to Eg5 (Transduction Laboratories, KY), α-tubulin (DM1A, Sigma, Germany), HEC1 (9G3.23, Novus Biologicals, CO) and centrin (J. L. Salisbury, Rochester, MN), a rabbit monoclonal antibody against survivin (71G4B7E, Cell Signaling Technology, MA), rabbit polyclonal antibodies against PRY1 (H-170, Santa Cruz, CA), phospho-Histone H3 (Ser10, Millipore, MA) and actin (I-19, Santa Cruz, CA), and a human antibody against Crest (ANA, Euroimmun, Germany). A rabbit polyclonal antibody against Cep164 and a sheep polyclonal antibody against BubR1 were kindly provided by E. A. Nigg (Basel, Switzerland) and S. S. Taylor (Manchester, UK), respectively.

### Immunofluorescence

The following fluorochrome-conjugated secondary antibodies were used: anti-mouse Alexa-488, anti-human Alexa-488 (Molecular Probes, OR), anti-mouse Cy3, anti-rabbit Cy3, and anti-sheep Cy3 (Jackson ImmunoResearch Laboratories, PA). For DNA counterstaining, DAPI contained in mounting medium (Vectashield, Vector Laboratories, CA) or Hoechst (Invitrogen) was used. Immunostained cells were examined using Axiovert 200 M (Zeiss) and LSM 710 (Zeiss, Zeiss Application Center, Heidelberg) equipped with LD-Plan neofluar 40x/0.6, EC-Plan Neofluar 40/1.30, EC-Plan Neofluar 100/1.30 and Plan-Apochromat 63x/1.4 obejctives (Zeiss). Images were processed with AxioVision (Zeiss), Zen 2008 (Zeiss), ImageJ (NIH), and Photoshop software (Adobe).

### Transfection

siRNAs were obtaind from either Dharmacon (siGENOME Set of 4 upgrades) or Eurofins MWG Operon and reverse transfected into the indicated cell lines as described above. SCC 114 cells were transiently transfected with pEGFP-C1-Bor1-141 and pEGFP-C1-Sur89-142 using Fugene 6 (Roche Applied Science) according to the manufacturer's instructions.

### Immunoblotting

Cell protein extracts were made by lysis of cells in an appropriate volume of RIPA buffer (50 mM Tris-HCl, pH 7.5, 150 mM NaCl, 1% Nonidet P40 (Genaxxon), 0.5% sodium deoxycholate, 0.1% SDS) supplemented with one Complete Protease Inhibitor Cocktail Tablet (Roche Diagnostics) per 50 ml of buffer, followed by mechanical homogenization and collection of the supernatant after 15 min centrifugation at 20,000g. Immunoblotting was performed according to standard protocols.

### Example 2: RNAi Screen for the Identification of Genes Required for Centrosomal Clustering

A genome-wide RNAi screen to uncover the molecular pathways involved in clustering of supernumerary centrosomes in human cancer cells was used (Fig. 1). For the screen the oral squamous cell carcinoma cell line SCC114 was used because >60% of cells contain extra centrosomes which are efficiently clustered into bipolar spindles in >95% of mitoses. A commercially available human RNAi library (Human siARRAY-Genome, Dharmacon) targeting 21,125 genes was used, with each gene being targeted by a siRNA pool consisting of four siRNAs directed against different regions of said gene (Fig. 1A). SCC114 cells were exposed to siRNAs for 48 hours. Subsequently, cells were fixed and stained for DNA and microtubules. Images were acquired using a 20x objective and a high-throughput automated fluorescence microscope (Fig. 1B). Per siRNA pool nine pictures were taken, thereby ensuring that representative areas of each incubate were covered.

By visual inspection of approximately 200,000 images, the percentage of multipolar spindles for each RNAi pool was scored. Two hundred mitotic cells per pool were analyzed and scored as a hit when a highly significant difference (more than three standard deviations) from negative controls was observed. When using these criteria and after thorough re-evaluation of each image-positive siRNA pool by direct microscopic analysis, the primary screen identified 133 candidates associated with a multipolar spindle phenotype (Fig. 1C). High-throughput RNAi screens typically produce false positives due to experimental errors and off-target effects. To minimize their number, a secondary screen was performed, with each hit from the primary screen being targeted by every single of the four siRNAs per siRNA pool in separate experiments (Fig. 1A). By using this strategy, 82 from the initial 133 genes were confirmed to induce spindle multipolarity in SCC114 cells (Fig. 1C). Among the validated genes, 22 (26.8%) play a role in mitosis, six (7.3%) are involved in cell adhesion, four (4.9%) are involved in proteolysis and ubiquitination, and 13 (15.9%) do not have a known function (Fig. 1D). To control for siRNA-mediated knockdown efficacy, quantitative RT-PCR was performed on 13 randomly selected genes from the list of validated genes and showed a knockdown efficacy of 86.9 ± 9.8% (range 96.5 ± 1.0% to 56.4 ± 0.6%; Fig. 2A). To further demonstrate knockdown at the protein level, Western blots were performed in a subset of cases with comparably weak knockdown efficacies at the mRNA level (Fig. 2B).

The genes identified in this screen to be required for centrosomal clustering which were not earlier implicated in this function are compiled in Table 1.

siRNA-mediated knockdown of several of these genes was confirmed to induce spindle multipolarity in an independent cell line (MDA-231).

### Example 3: Inhibition of Centrosomal Clustering Constitutes the Predominant Mechanism of Multipolar Spindle Formation in SCC114 Cells

To ascertain that spindle multipolarity after siRNA-induced knockdown of the genes identified in the screen is indeed caused by inhibition of centrosomal clustering and not due to the formation of acentrosomal spindle poles, SCC114 cells were co-immunostained with antibodies to α-tubulin and centrin as a centriolar marker, respectively, after exposure to siRNAs for 48 hours. Greater than 50% of spindle poles in cells with multipolar spindles contained centrin signals for 29 out of 32 siRNAs examined (90.6%), clearly indicating that spindle multipolarity in the majority of cases is not a consequence of acentrosomal spindle pole formation (Fig. 1E). In contrast, only 3 out of 32 siRNAs (9.4%) led to multipolar spindles with centrin signals at only two poles.

Multipolar mitoses by inhibition of centrosomal clustering should not arise in normal cells without supernumerary centrosomes. In keeping with this prediction, depletion of chromosomal passenger complex (CPC) and Ndc80 complex proteins, which were among the hits identified in the screen, did not lead to a significant induction of spindle multipolarity in normal diploid BJ fibroblasts. In contrast, depletion of proteins involved in cytokinesis which induce spindle multipolarity only after cytokinesis failure via duplication of the cellular centrosome content led to multipolarity to a certain extent in BJ fibroblasts as well. Also, knockdown of augmin complex components, whose disruption induces microtubule-dependent fragmentation of centrosomes, induces multipolar spindles in BJ fibroblasts. Fittingly, whereas after depletion of proteins involved in cytokinesis virtually all spindle poles contained centrin signals, knockdown of augmin complex components led to a significant fraction of acentriolar spindle poles.

**Table 1: Validated genes required for centrosomal clustering not earlier implicated in this function.**

| **Gene** | **Accession No.** | **Process** | **Function** | **Multipola rity (%)** |
|---|---|---|---|---|
| CEP164 | NM_014956.4 GI:115648141 | Mitosis | Primary cilium formation | 14.5 |
| ARL2 | NM_001667.2 GI:14861288 | Mitosis | GTP binding, MT binding | 14.1 |
| HEI-C | NM_138443.3 GI:224586869 | Mitosis | MT associated protein | 14.5 |
| SPC24 | NM_182513.2 GI:148747229 | Mitosis | Kinetochore component | 16.5 |
| CENP-T | NM_025082.3 GI:126722968 | Mitosis | Kinetochore component | 23.5 |
| MKLP1 | NM_004856.4 GI:20143965 | Mitosis | Cytokinesis | 52.6 |
| HIST1H2BO | NM_003527.4 GI:16306565 | Mitosis | Centromeric histone | 16 |
| HISTIH2BJ | NM_021058.3 GI:20336753 | Mitosis | Centromeric histone | 14.5 |
| FAM29A | NM_017645.3 GI:31377561 | Mitosis | MT binding | 24.3 |
| ADPGK | NM_031284.4 GI:187829199 | Metabolism | Glucokinase | 17.2 |
| ARSF | NM_004042.3 GI:31742481 | Metabolism | Arylsulfatase | 16.5 |
| MAP6 | NM_024871.2 GI:209915605 | Miscellaneous | MT binding | 15.5 |
| GOLM1 | NM_016548.2 GI:29550837 | Miscellaneous | Golgi transmembrane protein | 16.6 |
| STX5A | NM_003164.3 GI:94400931 | Miscellaneous | Vesicle transport, ESCRT | 14.5 |
| CACNA1F | NM_005183.2 GI:53832006 | Miscellaneous | Calcium channel protein | 16 |
| TXNL1 | NM_031284.4 GI:187829199 | Miscellaneous | Redox sensor, endocytosis | 15 |
| USP54 | NM_152586.3 GI:124001557 | Proteolysis | Ubiquitin-specitic peptidase | 15.5 |
| RGL2 | NM_004761.2 GI:21361071 | Signal transduction | Ras effector protein | 14.8 |
| MAP4K5 | NM_006575.3 GI:38570133 | Signal transduction | Protein kinase, stress response | 13.7 |
| TULP3 | NM_003324.3 GI:42544229 | Signal transduction | G protein signaling | 15 |
| GRP137 | NM_173481.2 GI:34222226 | Signal transduction | G protein-coupled receptor | 14 |
| ZNF354C | NM_014594.1 GI:30794503 | Transcription, translation | Transcriptional repressor, zink finger protein | 14.5 |
| MEPCE | NM_019606.4 GI:47271405 | Transcription, translation | Capping enzyme | 16 |
| MTF2 | NM_007358.3 GI:166706893 | Transcription, translation | Transcription factor | 14.4 |
| TNRC4 | NM_007185.3 GI:71164893 | Transcription, translation | pre mRNA splicing | 17 |
| POP1 | NM_015029.2 GI:225007647 | Transcription, translation | pre rRNA processing | 17 |
| SF3B1 | NM_012433.2 GI:54112116 | Transcription, translation | pre mRNA splicing | 15 |
| SNRPD2 | NM_004597.4 GI:29294622 | Transcription, translation | pre mRNA splicing | 14 |
| UPF3A | NM_023011.2 GI:18375523 | Transcription, translation | mRNA nuclear export | 14 |
| EIF3S6IP | NM_016091.2 GI:51093838 | Transcription, translation | Translation initiation factor | 14 |
| ZNF17 | NM_006959.2 GI:115494999 | Unknown | Zink finger protein | 16.5 |
| NACAD | XM_166571.6 GI:169170976 | Unknown | Unknown | 28 |
| C4ORF15 | NM_024511.5 GI:141802706 | Unknown | Unknown | 20 |
| C1ORF114 | NM_021179.1 GI:10880974 | Unknown | Unknown | 15.5 |
| C19ORF52 | NM_138358.2 GI:34328079 | Unknown | Unknown | 14.7 |
| ABHD8 | NM_024527.4 GI:145611433 | Unknown | Unknown | 14.5 |
| TM4SF18 | NM_138786.2 GI:142345050 | Unknown | Transmembrane protein | 14.5 |
| F8A1 | NM_012151.3 GI:56790933 | Unknown | Unknown | 14 |
| KIAA1191 | NM_020444.3 GI:119120886 | Unknown | Unknown | 14 |
| C20ORF149 | NM_024299.2 GI:34147371 | Unknown | Unknown | 14 |
| C11ORF4 | NM_020155.2 GI:21361824 | Unknown | Unknown | 14 |

## Claims

1. A compound modulating the function of a target gene in a cell, wherein the target gene is selected from the list consisting of CEP164, ARL2, HEI-C, SPC24, CENP-T, MKLP1, HIST1H2BO, HIST1H2BJ, FAM29A, ADPGK, ARSF, MAP6, GOLM1, STX5A, CACNA1F, TXNL1, USP54, RGL2, MAP4K5, TULP3, GRP137, ZNF354C, MEPCE, MTF2, TNRC4, POP1, SF3BI, SNRPD2, UPF3A, EIF3S6IP, ZNF17, NACAD, C4ORF15, C1ORF114, C19ORF52, ABHD8, TM4SF18, F8A1, KIAA1191, C20ORF149, and C11ORF4.

2. The compound of claim 1, wherein the compound negatively interferes with the function of the target gene.

3. The compound of any one of claims 1 or 2, wherein the compound is selected from a group consisting of an RNAi agent, a ribozyme, a DNAzyme, an inhibitory antibody, and an aptamer.

4. The compound of any one of claims 1 to 3, wherein said function of the target gene is mediation of centrosomal clustering.

5. A medicament comprising a compound of any one of claims 1 to 4 for the treatment or prevention of cancer.

6. The medicament of claim 5, wherein the cancer is selected from a list consisting of acute lymphoblastic leukemia, acute myeloid leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cance, testicular cancer, throat cancer, thymic carcinoma, thymoma, thyroid cancer, urethral cancer, uterine sarcoma, vaginal cancer, vulvar cancer, waldenström macroglobulinemia, and wilms tumor.

7. The medicament of claim 5, wherein the cancer is selected from a list consisting of multiple myeloma, leukemia, colon cancer, cervix carcinoma, pancreatic cancer, breast cancer, glioblastoma, and osteosarcoma.

8. The medicament of claim 6, wherein the cancer is a head and neck cancer.

9. Use of a compound of any one of claims 1 to 4 for the manufacture of a medicament for the treatment and / or prevention of cancer.

10. The use of claim 9, wherein the cancer is selected from the list defined in claim 6.

11. Use of a polypeptide encoded by a target gene selected from the group defined in claim 1 for the manufacture of a medicament for the treatment and / or prevention of cancer.

12. The use of claim 11, wherein the cancer is selected from the list defined in claim 6.

13. Method for the identification of a compound capable of inhibiting the function of a target gene in centrosomal clustering, comprising the steps of
a) expressing in a target cell a polynucleotide comprising a target gene selected from the group defined in claim 1,
b) contacting said target cell with a compound suspected of being able to inhibit the function of said target gene, and
c) determining the number of centrosomal clusters in said target cell, whereby a decrease in the number of centrosomal clusters after over-expression of said target gene is indicative for a compound capable of inhibiting the function of said target gene in centrosomal clustering.

14. Method for the identification of a compound capable of binding to a polypeptide encoded by a target gene as defined in claim 11 comprising the steps of
a) contacting said polypeptide with a compound suspected to bind to said polypeptide and
b) determining the amount of said compound bound to said polypeptide, whereby an increased binding compared to a reference is indicative for a compound capable of binding to said gene product.

15. A method for the identification of cancer cells in a sample comprising the steps of
a) contacting said sample with a diagnostic antibody directed against a polypeptide encoded by the polynucleotide as defined in claim 10 and
b) determining the amount of diagnostic antibody bound to said sample, whereby an increased binding compared to a reference is indicative for a cancer cell.

16. A method for the identification of cancer cells in a sample comprising the steps of
a. contacting said sample with a polynucleotide being capable of specifically hybridizing to the messenger RNA transcribed from a target gene selected from the list defined in claim 1 and
b. determining the amount of said polynucleotide bound to said sample, whereby an increased binding compared to a reference is indicative for a cancer cell.
